# EUROPÄISCHE PATENTSCHRIFT

(11) **EP 2 346 450 B1**
(45) Veröffentlichungstag und Bekanntmachung des Hinweises auf die Patenterteilung: **07.10.2015**
(21) Anmeldenummer: 09777613.2
(22) Anmeldetag: 03.08.2009
(51) Int. Cl.: A61F 2/90, D04C 1/06, A61F 2/88

(54) **MEDIZINISCHE VORRICHTUNG**
MEDICAL DEVICE
DISPOSITIF MÉDICAL

(30) Priorität: 05.08.2008 DE 102008036428; 23.09.2008 DE 102008048416
(43) Veröffentlichungstag der Anmeldung: 27.07.2011
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2009/005606
(87) Internationale Veröffentlichungsnummer: WO 2010/015370

(56) Entgegenhaltungen:
- WO-A-88/00813
- US-A- 4 743 251

## Beschreibung

Die Erfindung betrifft eine medizinische Vorrichtung gemäß dem Oberbegriff des Patentanspruchs 1. Eine medizinische Vorrichtung der eingangs genannten Art ist beispielsweise aus der US 6,258,115 B1 bekannt.

US 6,258,115 B1 offenbart einen Stent mit einer rotationssymmetrischen rohrförmigen Gitterstruktur, die aus einem Drahtgeflecht gebildet ist. Die Maschengröße des Drahtgeflechts ist bereichsweise unterschiedlich, so dass der Stent verschiedene Durchlässigkeiten aufweist. Vorzugsweise umfasst ein mittlerer Stentbereich größere Maschen, so dass der Stent beim Einsatz im Bereich einer Gefäßverzweigung einen ausreichenden Blutfluss in ein Nebengefäß ermöglicht.

Die Porosität bzw. Durchlässigkeit der Stentbereiche wird unter anderem durch den Flechtwinkel bestimmt. Der Flechtwinkel ist als derjenige Winkel definiert, der zwischen einem spiralförmig um die Rotationsachse des Stents gewundenen Draht in Draufsicht mit einer zur Rotationsachse parallelen Geraden bzw. einer Projektion der Rotationsachse in die Umfangsebene gebildet ist. Beim Vergleich der Flechtwinkel zweier Drähte des Stents, wobei die zu vergleichenden Winkel in derselben Querschnittsebene des Stents betrachtet werden, ist festzustellen, dass die beiden Flechtwinkel gleich sind. Eine Variation des Flechtwinkels zur Änderung der Durchlässigkeitseigenschaften des Stents erfolgt nur in axialer Richtung des Stents. Dies ist insbesondere bei Betrachtung der Kreuzungs- bzw. Knotenpunkte ersichtlich, an denen sich zwei spiralförmig um die Rotationsachse verlaufende Drähte kreuzen. Die beiden Drähte weisen bezüglich einer zur Rotationsachse parallelen Geraden, die durch den Kreuzungsbereich verläuft, denselben Winkel auf.

Allgemein werden Vorrichtungen mit einer rotationssymmetrischen Gitterstruktur, die zwei spiralförmig um eine gemeinsame Rotationsachse gewundene Drahtelemente umfassen, d.h. Geflechte oder Coils, in der Medizintechnik zur Verstärkung von Zufuhrsystemen, beispielsweise Katheter oder Schläuche, eingesetzt. Derartige Zufuhrsysteme werden unter Anderem für die Zufuhr von Stoffen, Medikamenten oder Kontrastmitteln in oder aus dem Körper oder zur Diagnose, insbesondere zur Messung von Temperatur oder Druck, eingesetzt. Eine weitere Anwendungsmöglichkeit derartiger Geflechte oder Coils betrifft die Verstärkung von Schläuchen zur Endoskopie. Das in die meist aus Kunststoff bestehenden Schläuche eingearbeitete Drahtgerüst dient dabei zur Stabilisierung des Schlauches. Insbesondere soll dabei das Knicken des Schlauches in engen Biegeradien verhindert werden.

Bekannte Drahtgeflechte bzw. Drahtspiralen haben die Eigenschaft, dass sich der Querschnittsdurchmesser der rotationssymmetrischen Gitterstruktur in Abhängigkeit einer Längenänderung des Drahtgeflechts verändert. So führt eine Verkürzung des Geflechts bzw. Coils zu einer Aufweitung des Querschnittsdurchmessers, wohingegen eine Verlängerung des Geflechts bzw. Coils eine Reduktion des Querschnittsdurchmessers bewirkt. Bei kunststoffummantelten Drahtstrukturen mit kleiner Wandstärke, beispielsweise bei einem mit einem Drahtgeflecht verstärkten Katheter, führt ein Schieben des Katheters im Blutgefäß zu einer Verkürzung der Katheterlänge, wodurch der Querschnittsdurchmesser des Katheters erhöht wird. Dadurch kommt der Katheter mit der Gefäßwand in Kontakt, d.h. die Reibung zwischen Katheter und Gefäßwand wird erhöht, so dass ein weiteres Vorschieben des Katheters verhindert oder zumindest erschwert wird. Dabei besteht das Risiko, dass der aufgeweitete Katheter das Gefäß verschließt bzw. die Blutströmung reduziert, was mit gesundheitlichen Folgen für den Patienten verbunden sein kann.

Umgekehrt verhält es sich beim Ziehen des Katheters, insbesondere des Drahtgeflechts bzw. Coils innerhalb des Katheters. Das Ziehen bewirkt eine Verlängerung des Katheters, wodurch der Querschnittsdurchmesser des Schlauches reduziert wird. Bei Aspirationskathetern bewirkt eine derartige Reduktion der lichten Weite beispielsweise einen Verlust an Saugkraft. Bei Kathetern, die zum Einbringen von Implantaten in den menschlichen Körper eingesetzt werden, wird auf das im Katheter geführte Implantat ein radialer Druck aufgebracht, der zu einer Beschädigung des Implantats führen kann.

Grundsätzlich bewirkt die beschriebene Längenänderung insbesondere bei langen Kathetersystemen eine erschwerte Positionierung der Katheterspitze am gewünschten Ziel bzw. Behandlungsort. Das Drahtgeflecht bzw. die spiralförmig gewundenen Drähte wirken dabei federartig, so dass ein Schieben bzw. Ziehen des Katheters auf Anwenderseite, d.h. außerhalb des Körpers, intrakorporal an der Katheterspitze nicht identisch umgesetzt wird.

Zwar wird die Drahtstruktur durch die Einbettung in einen Kunststoff bzw. die Umhüllung mit einem Kunststoff zumindest teilweise versteift, wenn die Wandstärke der Umhüllung entsprechend groß ist. Dabei nimmt allerdings auch die Flexibilität des Systems ab, da sich der Kunststoff wegen der verhältnismäßig hohen Wandstärke nur schlecht ausdehnt oder komprimiert. Insbesondere bei kleinen Biegeradien besteht das Risiko, dass die Kunststoffschicht trotz der stabilisierenden Wirkung des Drahtgeflechts einknickt, wodurch die Funktionalität des Systems, insbesondere bei Kathetern, beeinträchtigt wird.

Einen besonderen Anwendungsfall für bekannte drahtverstärkte Schläuche stellt der Stabilizer dar, der dazu dient, Implantate aus einem Katheter in ein Gefäß heraus zu schieben. Dabei befindet sich der Stabilizer, d.h. ein drahtverstärkter Schlauch, innerhalb des Katheterschlauches. Derartige Kathetersysteme sind vorzugsweise relativ klein, um in kleinen Gefäßen, beispielsweise Herzkranzgefäßen oder intrakraniellen Gefäßen, einsetzbar zu sein. Eine durch das Schieben des Stabilizers bewirkte Durchmessererhöhung führt bei derartig kleinen Abmessungen und Toleranzen zu einer Blockade des Systems, so dass der Stabilizer aufgrund der erhöhten Reibung zwischen der Innenwand des äußeren Katheterschlauches und dem Stabilizer selbst, nicht weiter in Richtung des Behandlungsortes geschoben werden kann. Um die Durchmesseränderung beim Schieben des Stabilizers zu verhindern, besteht die Möglichkeit, das Drahtgeflecht unterstützend zu verstärken, indem die Wandstärke der Kunststoffbeschichtung erhöht wird. Dadurch wird der gesamte Durchmesser des Kathetersystems vergrößert, wodurch eine Behandlung kleiner Gefäße verhindert wird.

Der Erfindung liegt daher die Aufgabe zu Grunde, eine Möglichkeit zu schaffen, die radiale Stabilität einer medizinischen Vorrichtung zu beeinflussen.

Erfindungsgemäß wird diese Aufgabe durch den Gegenstand des Patentanspruchs 1 gelöst.

Die Erfindung beruht auf dem Gedanken, eine medizinische Vorrichtung, aufweisend eine rotationssymmetrische Gitterstruktur mit wenigstens zwei spiralförmig um eine gemeinsame Rotationsachse R gewundenen Drahtelementen anzugeben, die mit einer gemeinsamen, senkrecht zur Rotationsachse R angeordneten Ebene L jeweils einen ersten und zweiten Schnittpunkt S', S" bilden, wobei durch den ersten Schnittpunkt S' eine erste Gerade R' und durch den zweiten Schnittpunkt S" eine zweite Gerade R" verlaufen, die jeweils parallel zur Rotationsachse R angeordnet sind und mit jeweils einem der beiden Drahtelemente einen spitzen Winkel einschließen. Dabei sind die beiden Winkel unterschiedlich.

Die Konfiguration der Flechtwinkel, d.h. der Winkel α' und α", bezieht sich auf den Ruhezustand der Vorrichtung. Die Bestimmung des Flechtwinkels wird bei der Vorrichtung im entspannten Zustand, d.h. ohne Einwirkung äußerer Kräfte, vorgenommen. Vorzugsweise ist die Vorrichtung zur Bestimmung der Winkel in axialer Richtung geradlinig ausgerichtet.

Die Erfindung betrifft somit eine neuartige Flechtkonfiguration bzw. Ausrichtung der Drahtelemente für medizinische Vorrichtungen, wobei der Winkel zwischen einem ersten Drahtelement und einer senkrechten Projektion der Rotationsachse R auf die Umfangsebene der Gitterstruktur gegenüber dem Winkel zwischen einem zweiten Drahtelement und einer weiteren senkrechten Projektion der Rotationsachse R auf die Umfangsfläche der Gitterstruktur unterschiedlich ist. Der Vergleich der Winkel erfolgt auf derselben axialen Höhe der Gitterstruktur, d.h. in einer senkrecht zur Rotationsachse R orientierten Querschnittsebene L der Gitterstruktur. Auf diese Weise wird eine bezüglich der Winkel bzw. Flechtwinkel asymmetrische Gitterstruktur bereitgestellt. Bei einer Durchmesser- oder Längenänderung verhalten sich separate Drahtspiralen, die verschiedene Winkel aufweisen, unterschiedlich. Dieser Unterschied bewirkt bei einer Kombination der Drahtelemente mit unterschiedlichen Winkeln, die in spiralförmig gewundener Weise die Gitterstruktur bilden, dass sich die Drahtelemente gegenseitig in ihrer Bewegungsfreiheit blockieren, so dass eine Durchmesseränderung verhindert wird. Auf diese Weise wird eine dimensions- bzw. formstabile Struktur bereitgestellt. Beim Einsatz der erfindungsgemäßen medizinischen Vorrichtung zur Verstärkung von medizinischen Schläuchen, beispielsweise Kathetern, wird ein Knicken oder Kollabieren erschwert, da durch die Stabilisierung des Schlauches durch das asymmetrische Geflecht eine Durchmesseränderung vermieden wird. Im Rahmen der Erfindung bezeichnet ein spitzer Winkel einen Winkel, der größer als 0° und weniger als 90° beträgt.

Bei einer bevorzugten Ausführungsform der erfindungsgemäßen medizinischen Vorrichtung umfassen alle Drahtelemente der Gitterstruktur dasselbe inelastische Material, so dass die Gitterstruktur im Wesentlichen eine starre, unveränderliche bzw. stabile Geometrie aufweist. Auf diese Weise wird der Durchmesser der Gitterstruktur bzw. allgemein deren Form nicht nur durch die Flechtkonfiguration bzw. die Geometrie, sondern auch durch die Werkstoffeigenschaften festgelegt. Durch die starre Struktur und die damit verbundene Längen- und Durchmesserstabilität wird sichergestellt, dass beispielsweise ein mit Hilfe der medizinischen Vorrichtung verstärkter Katheter bzw. ein als die medizinische Vorrichtung ausgebildeter Katheter exakt positionierbar ist.

Bei einer alternativen Ausführungsform umfassen alle Drahtelemente der Gitterstruktur dasselbe elastische Material, so dass die rotationssymmetrische Gitterstruktur im Wesentlichen eine veränderbare Geometrie aufweist. Durch das elastische Material wird erreicht, dass eine gezielte Durchmesser- und Längenänderung der medizinischen Vorrichtung möglich ist. Das ist beispielsweise beim Einsatz der medizinischen Vorrichtung als Stent interessant, da auf diese Weise eine Rückstellkraft in radialer Richtung einstellbar bzw. verstärkbar ist, durch die die medizinische Vorrichtung bzw. der Stent im implantierten Zustand radial gegen die Gefäßwand gedrückt und somit verankert wird. Ferner erlauben die elastischen Drahtelemente, dass die medizinische Vorrichtung einer Bewegung der Gefäßwand, beispielsweise durch den Pulsschlag, folgen kann.

Vorzugsweise beträgt der Winkelunterschied zwischen dem ersten Winkel und dem zweiten Winkel wenigstens 2°, insbesondere wenigstens 5°, insbesondere wenigstens 8°, , insbesondere wenigstens 10°, insbesondere wenigstens 20°, insbesondere wenigstens 30°, insbesondere wenigstens 40°, insbesondere wenigstens 45°, insbesondere wenigstens 50°, insbesondere wenigstens 60°, insbesondere wenigstens 70°, insbesondere wenigstens 90°. Im Allgemeinen wird die radiale Stabilität der medizinischen Vorrichtung durch die Größe des Winkelunterschieds zwischen dem ersten und dem zweiten Winkel bzw. durch den Grad der Asymmetrie bestimmt. Je größer der Winkelunterschied ist, desto stärker blockieren sich die einander zugeordneten asymmetrisch verlaufenden Drähte bzw. Drahtelemente.

Ferner kann der Winkelunterschied zwischen dem ersten Winkel und dem zweiten Winkel höchstens 50°, insbesondere höchstens 45°, insbesondere höchstens 40°, insbesondere höchstens 30°, insbesondere höchstens 20°, insbesondere höchstens 10°, insbesondere höchstens 8°, insbesondere höchstens 6°, insbesondere höchstens 4°, insbesondere höchstens 2°, betragen. Die für eine Formänderung bzw. Längendehnung der Drahtelemente erforderliche Kraft wird mit zunehmendem Winkelunterschied größer. Durch eine geeignete Wahl des Elastizitätsmoduls der Drahtelemente kann im Zusammenhang mit dem Winkelunterschied gezielt die Formstabilität bzw. eine radiale Rückstellkraft eingestellt werden. Je größer der Elastizitätsmodul, also je schwächer die Dehnbarkeit der Drahtelemente ist, und/oder je größer der Winkelunterschied ist, umso formstabiler ist das Gittergeflecht. Je kleiner der Winkelunterschied ist, umso dehnbarer ist eine rohrförmige Gitterstruktur, da die spiralförmig gewundenen Drahtelemente mit einem flachen Winkel eine höhere axiale Längenänderung zulassen. Die Obergrenze des Winkelunterschieds von 10°, insbesondere von 8°, insbesondere von 6°, insbesondere von 4°, insbesondere von 2°, ist für medizinische Zwecke besonders geeignet, da mit relativ geringen äußeren Kräften, beispielsweise beim Crimpen oder im implantierten Zustand im Gefäß, eine Längendehnung der Drähte und somit die Rückstellkraft bewirkt wird. Kleinere Winkelunterschiede als 2° sind möglich.

Wenn die Einstellung einer die Radialkraft verstärkenden Rückstellkraft im Vordergrund steht, beispielsweise bei einem Stent oder anderen Implantat, ist eine gewisse Dehnbarkeit bzw. Längenverformbarkeit der Drahtelemente erforderlich. Die Drahtelemente sind elastisch. Geeignete Materialien sind in der Anmeldung offenbart. Durch die sich in ihrer Bewegungsfreiheit gegenseitig blockierenden asymmetrischen Drahtelemente wird durch die Beaufschlagung mit einer äußeren Kraft eine Längenänderung zumindest der Drahtelemente mit flacherem Flechtwinkel erzeugt, die dadurch eine Rückstellkraft auf das Geflecht ausüben, die die Radialkraft verstärkt. Hierfür ist ein Flechtwinkelunterschied im Bereich von 2° bis 10° vorteilhaft, da zur Erzeugung der Rückstellkraft eine relativ geringe, für medizinische Zwecke geeignete äußere Kraft ausreicht.

Der erste Winkel kann kleiner als 45°, insbesondere kleiner als 40°, insbesondere kleiner als 20° sein.

Der zweite Winkel kann größer als 2°, insbesondere größer als 3°, insbesondere größer als 5°, insbesondere größer als 7°, insbesondere größer als 45°, insbesondere größer als 50°, insbesondere größer als 70°, betragen.

Des Weiteren kann das Verhältnis der Anzahl der ersten Drahtelemente, d.h. der Drahtelemente mit dem ersten Winkel und der Anzahl der zweiten Drahtelemente, d.h. der Drahtelemente mit dem zweiten Winkel, höchstens 1:1, insbesondere höchstens 1:2, insbesondere höchstens 1:4, insbesondere höchstens 1:6, insbesondere höchstens 1:8, insbesondere höchstens 1:12, insbesondere höchstens 1:24, betragen. Vorzugsweise weisen die ersten Drahtelemente einen kleineren Winkel auf als die zweiten Drahtelemente. Aufgrund der asymmetrischen Geometrie werden die ersten Drahtelemente bzw. generell Drahtelemente mit einem relativ kleinen Winkel stärker gedehnt als Drahtelemente, die in einem größeren Winkel zur Rotationsachse R angeordnet sind, und erzeugen dadurch eine Rückstellkraft, so dass bei Drahtstrukturen mit geringer Radialkraft diese erhöht wird. Dadurch, dass die Anzahl der ersten Drahtelemente, die einen kleineren Winkel aufweisen, gegenüber der Anzahl der zweiten Drahtelemente kleiner ist, kann die Rückstellkraft der Gitterstruktur fein eingestellt werden.

Grundsätzlich ist eine Dehnung der Drahtelemente bei kleinen Winkeldifferenzen zwischen den ersten und zweiten Drahtelementen möglich. Im Unterschied zu symmetrischen Flechtkonfigurationen weisen beispielsweise rohrförmige Gitterstrukturen, insbesondere Stents, durch die asymmetrische Anordnung der Drahtelemente eine höhere Radialkraft auf, da die Drahtelemente mit kleinerem Winkel Drahtspiralen bilden, die mit einer geringeren Kraft in axialer Richtung auseinandergezogen bzw. gedehnt werden können. Wenn der Unterschied zwischen dem ersten und dem zweiten Winkel α', α" klein ist, insbesondere im Bereich von 2° bis 10°, sind die für die Dehnung der Drahtelemente erforderlichen äußeren Kräfte so gering, dass eine derart gestalteten Gitterstruktur für medizinische Anwendungen geeignet ist. Die die Dehnung bewirkenden äußeren Kräfte entstehen beispielweise bei der Überführung einer rohrförmigen Gitterstruktur bzw. eines Stents vom expandierten Zustand in einen komprimierten Zustand, d.h. beim Crimpen des Stents. Auch im implantierten Zustand ist der Stent zumindest teilweise komprimiert.

Bei einer bevorzugten Ausführungsform der erfindungsgemäßen medizinischen Vorrichtung ist wenigstens ein Stabilisierungsabschnitt vorgesehen, der sich zumindest bereichsweise in axialer Richtung entlang der Gitterstruktur erstreckt, wobei der erste Winkel entlang des Stabilisierungsabschnitts stets größer oder stets kleiner als der zweite Winkel ist. Auf diese Weise kann erreicht werden, dass bereichsweise eine Aufweitung oder Komprimierung der Gitterstruktur möglich ist, nämlich in den außerhalb des Stabilisierungsabschnitts angeordneten Abschnitten, während der Stabilisierungsabschnitt einen konstanten, unveränderbaren Durchmesser aufweist.

Der Stabilisierungsabschnitt kann einen axial mittleren Abschnitt und/oder axialen Endabschnitt der Gitterstruktur bilden. Durch die Bildung eines axial mittleren Abschnitts als Stabilisierungsabschnitt kann die Vorrichtung beispielsweise als Implantat, insbesondere Stent, in ein Gefäß eingesetzt werden, wobei der Stabilisierungsabschnitt ein Kollabieren des Gefäßes verhindert bzw. die Radialkraft zur Ausweitung des Gefäßes erhöht, da im Stabilisierungsabschnitt eine Durchmesseränderung verhindert bzw. die Radialkraft erhöht ist, wohingegen in den Endbereichen durch eine dort mögliche Durchmesseränderung die medizinische Vorrichtung der Bewegung der Gefäßwand, beispielsweise bei Pulsation, folgen kann (Compliance). Dadurch können stabile und einfach fixierbare künstliche Gefäße bzw. Gefäßprothesen bereitgestellt werden. Ferner können medizinische Vorrichtungen einen ersten axialen Endabschnitt aufweisen, der ein asymmetrisches Geflecht aufweist, und einen zweiten axialen Endabschnitt, der eine symmetrische bzw. flexible Flechtkonfiguration umfasst. Der erste, stabilisierte Endabschnitt ermöglicht ein einfaches Schieben der Vorrichtung, wohingegen der zweite, flexible Endabschnitt sich ausweiten und eine entsprechend gewünschte Funktion erfüllen kann.

Die medizinische Vorrichtung kann zwei Stabilisierungsabschnitte aufweisen, die jeweils einen axialen Endabschnitt der Gitterstruktur bilden. Die Anordnung des Stabilisierungsabschnitts in beiden Endabschnitten der Gitterstruktur hat den Vorteil, dass die Endabschnitte eine stabilisierende Funktion haben und ein mittlerer Abschnitt beispielsweise eine Flechtkonfiguration aufweist, die eine radiale Aufweitung bzw. Komprimierung erlaubt. Der Stabilisierungsabschnitt kann sich über die gesamte Gitterstruktur erstrecken.

Besonders bevorzugt ist eine Ausführungsform der medizinischen Vorrichtung, die zwei Stabilisierungsabschnitte aufweist, die jeweils einen axialen Endabschnitt der Gitterstruktur bilden. Beispielsweise können dadurch besonders stabile und einfach fixierbare künstliche Gefäße bzw. Gefäßprothesen bereitgestellt werden. Vorzugsweise variiert der erste Winkel und/oder der zweite Winkel zumindest abschnittsweise entlang der Gitterstruktur, insbesondere entlang einer parallel zur Rotationsachse R verlaufenden Geraden R', R". Diese Ausgestaltung ist vorteilhaft, da die Eigenschaften der Gitterstruktur entlang der Rotationsachse R änderbar, insbesondere kontinuierlich änderbar bzw. einstellbar sind.

Bei einer bevorzugten Ausführungsform der erfindungsgemäßen medizinischen Vorrichtung ist die rotationssymmetrische Gitterstruktur im Wesentlichen rohrförmige, insbesondere stentartig, ausgebildet. Auf diese Weise eignet sich die Vorrichtung besonders zur Verstärkung bzw. Stabilisierung von Schläuchen mit einem konstanten Durchmesser. Ferner wird durch die rohrförmige Gitterstruktur erreicht, dass die Vorrichtung als Implantat zur Stützung von Körpergefäßen, insbesondere Blutgefäßen, einsetzbar ist.

Vorzugsweise sind die Drahtelemente entlang der Gitterstruktur zumindest abschnittsweise coilartig gewunden. Durch die coilartige bzw. spiralförmige Anordnung der Drahtelemente ist eine einfache Herstellung der erfindungsgemäßen Vorrichtung möglich.

Die Drahtelemente können entlang der Gitterstruktur zumindest abschnittsweise miteinander verflochten sein. Bei einer Flechtstruktur wird jeweils ein Drahtelement über und unter weitere Drahtelemente geführt, so dass durch die Reibungskraft zwischen den Drahtelementen die Stabilität und Steifigkeit der Gitterstruktur weiter erhöht wird.

Ferner können die Drahtelemente zumindest an einem axialen Ende oder in einem Kreuzungsbereich der Gitterstruktur miteinander verbunden, insbesondere form-, kraft- und/oder stoffschlüssig, insbesondere verklebt oder verschweißt, sein. Beispielsweise können die Drahtelemente an einem axialen Ende der Gitterstruktur miteinander verbunden sein derart, dass die Gitterstruktur im Wesentlichen aus einem zusammenhängenden Draht gebildet ist, der an den axialen Enden jeweils umgelenkt und in die Gegenrichtung geführt ist. Auf diese Weise werden offene Drahtenden an den axialen Enden der Gitterstruktur vermieden und beispielsweise beim Einsatz der Vorrichtung als Stent die Verletzungsgefahr reduziert. Ferner ermöglicht eine Verbindung der Drahtelemente untereinander, insbesondere in einem Kreuzungsbereich, eine weitere, verbesserte Stabilisierung der Gitterstruktur, da ein Gleiten der sich kreuzenden Drahtelemente aufeinander vermieden wird. Vorzugsweise sind die Drahtelemente an den axialen Enden des Stabilisierungsabschnitts bzw. der Gitterstruktur verbunden, so dass die Blockierung der Drahtelemente gewährleistet ist.

Bevorzugterweise begrenzt der Kreuzungsbereich, in dem die Drahtelemente miteinander verbunden sind, den Stabilisierungsabschnitt. Dadurch wird verhindert, dass eine Relativbewegung der Drahtelemente untereinander, die in Abschnitten der Gitterstruktur, die keinen Stabilisierungsbereich bilden, möglich ist, in den Stabilisierungsabschnitt, insbesondere den Abschnitt mit der asymmetrischen Flechtkonfiguration, übertragen werden. Die Kreuzungsbereiche, in denen die Drahtelemente miteinander verbunden sind, stellen eine Begrenzung des Stabilisierungsabschnitts dar und ermöglichen eine herstellungsbedingt einfach festlegbare Trennung zwischen dem Stabilisierungsabschnitt und weiteren Abschnitten der Gitterstruktur. Es ist auch möglich, dass im Bereich des asymmetrischen Geflechts durch die Geometrie bzw. durch die reibschlüssige Verbindung in den Kreuzungsbereichen eine Relativbewegung der Drahtelemente zueinander verhindert wird, ohne dass die Kreuzungsbereiche bzw. Knotenpunkte zusätzlich fixiert sind, da das Geflecht generell eine Eigenstabilität besitzt..

Bei einer bevorzugten Ausführungsform der erfindungsgemäßen, medizinischen Vorrichtung umfassen die Drahtelemente ein Formgedächtnismaterial und/oder ein pseudoelastisches Material, insbesondere eine Nickel-Titan-Legierung. Derartige Materialien weisen eine hohe Beständigkeit und Biokompatibilität auf und ermöglichen außer dem, dass im Rahmen der pseudoelastischen Eigenschaften eine festlegbare Durchmesseränderung bzw. radial wirkende Kraftkomponente der Gitterstruktur einstellbar ist.

Alternativ können die Drahtelemente einen Kunststoff, insbesondere Polyester, Polyamid, Polypropylen oder Polyethylen, insbesondere HDPE oder UHMWPE umfassen. Auf diese Weise kann die Gitterstruktur mit einer elastisch veränderbaren Geometrie versehen werden, die durch die elastischen Eigenschaften des verwendeten Kunststoffs erreicht wird. Insbesondere kann durch entsprechende Wahl des Kunststoffes eine Verformbarkeit und Durchmesseränderung bzw. Radialkraftkomponente der Gitterstruktur eingestellt werden.

Bei einer bevorzugten Ausführungsform ist eine flexible Umhüllung vorgesehen, die sich zumindest abschnittsweise in Umfangsrichtung und/oder axialer Richtung entlang der Gitterstruktur erstreckt. Die Umhüllung kann auf einem äußeren oder inneren Umfang der Gitterstruktur angeordnet sein oder die Drahtelemente vollständig umhüllen derart, dass die Gitterstruktur zumindest abschnittsweise vollständig in die Umhüllung eingebettet ist. Durch die Kombination der asymmetrisch geflochtenen Gitterstruktur mit einer flexiblen Umhüllung werden insbesondere bei rohrförmigen Gitterstrukturen schlauchartige Gebilde bereitgestellt, die eine besonders hohe Stabilität und Festigkeit in radialer Richtung aufweisen, wobei die Schläuche ferner eine ausreichende Flexibilität aufweisen, um in axialer Richtung gebogen bzw. geknickt zu werden. Auf diese Weise können beispielsweise Katheterschläuche bereitgestellt werden, die im Wesentlichen in jedem Betriebszustand einen gleichmäßigen Durchmesser aufweisen und gleichzeitig ausreichend flexibel sind, um durch Krümmungen von Körpergefäßen manövriert zu werden. Die flexible Umhüllung umfasst vorzugsweise einen Kunststoff, insbesondere Polyurethan, Silikon oder Teflon.

Bei einer weiteren bevorzugten Ausführungsform der Erfindung weisen mehrere erste Drahtelemente jeweils den ersten Winkel α' auf (erster Flechtwinkel) und bilden untereinander eine erste symmetrische Struktur. Mehrere zweite Drahtelemente weisen den zweiten Winkel α" auf (zweiter Flechtwinkel) und bilden untereinander eine zweite symmetrische Struktur. Die erste Struktur und die zweite Struktur sind überlagert derart, dass die ersten und zweiten Drahtelemente mit unterschiedlichen ersten und zweiten Winkeln α', α" einander zugeordnet sind. Bei dieser Ausführungsform sind also zwei verschiedene Strukturen vorgesehen, die jeweils in sich symmetrisch ausgebildet sind, also jeweils innerhalb der Struktur denselben Flechtwinkel aufweisen. Die beiden Strukturen sind miteinander kombiniert und wirken kräftemäßig zusammen, wobei die Drahtelemente der verschiedenen Strukturen, also die ersten und zweiten Drahtelemente unterschiedliche Flechtwinkel aufweisen. Durch die Überlagerung der ersten und zweiten Struktur wird insgesamt eine asymmetrische Gitterstruktur gebildet, die zwei in sich symmetrische und im Hinblick auf die Flechtwinkel verschiedene Unterstrukturen umfasst.

Es ist auch möglich, dass mehr als zwei Strukturen vorgesehen sind, die in sich symmetrisch sind und, miteinander kombiniert, insgesamt eine asymmetrische Gesamtstruktur ergeben, wobei die Gesamtstruktur unterschiedliche, d. h. drei oder mehr unterschiedliche Flechtwinkel aufweist. Die Strukturen sind überlagert und miteinander verbunden, bzw. wirken zusammen derart, dass eine Kraftübertragung zwischen den Drahtelementen bzw. den Unterstrukturen möglich ist. Die Unterstrukturen sind vorzugsweise miteinander verflochten oder verwoben. Bevorzugt ist eine Verbindung der Unterstrukturen in den Kreuzungsbereichen und/oder an den axialen Enden der Gitterstruktur und/oder an den Grenzen eines Stabilisierungsabschnitts.

Für die Asymmetrie des Gesamtgeflechts ist es ausreichend, wenn eine erste und zweite symmetrische Unterstruktur zusammenwirken, deren Flechtwinkel verschieden sind. Weitere in sich symmetrische drite, vierte oder mehr Unterstrukturen, die entweder überlagert oder in Längsrichtung nachgeordnet sind, können denselben Flechtwinkel wie die erste oder zweite Unterstruktur oder einen von den Flechtwinkeln der ersten und zweiten Unterstruktur verschiedenen Flechtwinkel aufweisen.

Der Vorteil der aus verschiedenen Unterstrukturen gebildeten Vorrichtung besteht darin, dass die die Vorrichtung eine besonders hohe Stabilität gegenüber Torsionskräften aufweist.

Bezüglich des Herstellungsverfahrens der medizinischen Vorrichtung gemäß Anspruch 1 beruht die Erfindung auf dem Gedanken, wenigstens zwei Drahtelemente spiralförmig um eine gemeinsame Rotationsachse zu binden, derart, dass die Drahtelemente in zumindest einer senkrecht zur Rotationsachse R angeordneten Ebene L mit einer parallel zur Rotationsachse R verlaufenden Geraden R', R" jeweils unterschiedliche spitze Winkel einschließen. Besonders bevorzugt ist das Flechten oder Winden der Drahtelemente mit einer Textilmaschine, insbesondere Spinn- oder Webmaschine.

Die Erfindung wird im Folgenden anhand von Ausführungsbeispielen unter Bezug auf die beigefügten schematischen Zeichnungen näher erläutert. Darin zeigen
- Fig. 1: die Anordnung zweier Drahtelemente der medizinischen Vorrichtung gemäß einem bevorzugten Ausführungsbeispiel;
- Fig. 2a, 2b: eine Flechtkonfiguration einer erfindungsgemäßen, medizinischen Vorrichtung gemäß einem weiteren Ausführungsbeispiel;
- Fig. 3a, 3b: eine weitere Flechtkonfiguration der erfindungsgemäßen, medizinischen Vorrichtung nach einem weiteren Ausführungsbeispiel;
- Fig. 4a, 4b: eine perspektivische Ansicht einer erfindungsgemäßen, medizinischen Vorrichtung nach einem weiteren, bevorzugten Ausführungsbeispiel; und
- Fig. 5: eine Detailansicht einer Flechtkonfiguration für eine erfindungsgemäße medizinische Vorrichtung gemäß einem weiteren Ausführungsbeispiel.

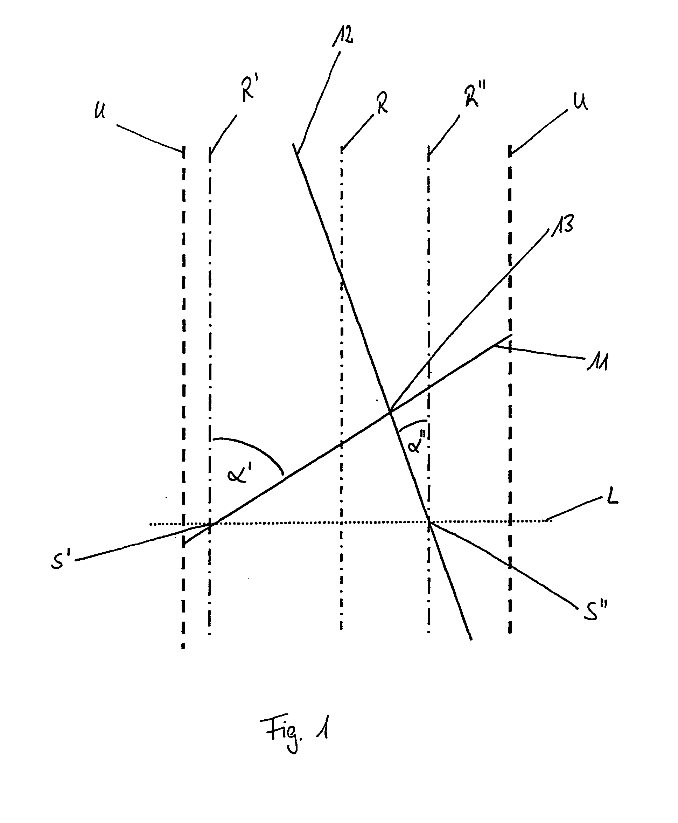

In Fig. 1 ist beispielhaft die Anordnung zweier Drahtelemente 11, 12 gezeigt, wobei insbesondere die Geraden R', R", die Ebene L sowie die durch die Geraden R', R" und die Ebene L gebildeten Schnittpunkte S', S" dargestellt sind. Die Ebene L und die Geraden R', R"sind imaginäre Bezugslinien bzw eine imaginäre Bezugsfläche zur Bestimmung des Flechtwinkels. Aus Gründen der Übersichtlichkeit sind in Fig. 1 nur zwei Drahtelemente 11, 12 dargestellt. Grundsätzlich kann die Gitterstruktur 10 mehrere Drahtelemente 11, 12 umfassen. Die Gitterstruktur 10 ist in der Darstellung gemäß Fig. 1 im aufgeklappten Zustand gezeigt, d.h. der Abstand zwischen den Begrenzungslinien U entspricht dem Umfang der Gitterstruktur 10. Die Drahtelemente 11, 12 überlappen sich und bilden dabei einen Kreuzungsbereich 13. Die gemäß Fig. 1 rohrförmige Gitterstruktur ist bezüglich einer Rotationsachse R rotationssymmetrisch ausgebildet. Eine senkrecht zur Rotationsachse R angeordnete Querschnittsebene L bildet mit den Drahtelementen 11, 12 jeweils einen Schnittpunkt S', S". Durch die Schnittpunkte S', S" verläuft jeweils eine Gerade R', R", die parallel zur Rotationsachse R und auf der Umfangsfläche der Gitterstruktur 10 angeordnet ist. Der zwischen dem ersten Drahtelement 11 und der ersten Geraden R' im Bereich des ersten Schnittpunktes S' gebildete Winkel α' entspricht dabei dem Flechtwinkel des ersten Drahtelements 11. Dasselbe gilt für den zweiten Winkel α", der im Bereich des zweiten Schnittpunkts S" durch den Winkel zwischen dem zweiten Drahtelement 12 und der zweiten Geraden R" gebildet ist. Der zweite Winkel α" entspricht dem Flechtwinkel des zweiten Drahtelements 12. Zum Vergleich der Winkel α', α" der beiden Drahtelemente 11, 12 werden grundsätzlich die spitzen Winkel herangezogen, d.h. Winkel mit einem Wert, der größer als 0° und kleiner als 90° beträgt. Es könnte auch jeweils der stumpfe Winkel verglichen werden. Es kommt also darauf an, dass die einander entsprechenden Winkel, d. h. jeweils die spitzen bzw. die stumpfen Winkel miteinander verglichen werden.

Wie in Fig. 1 erkennbar, sind die beiden Winkel α', α" unterschiedlich groß. Konkret ist der Winkel α' größer als der Winkel α". Das Drahtelement 12 mit dem Winkel α" hat demnach eine größere Steigung als das Drahtelement 11 mit dem Winkel α', wobei die Steigung einer entlang der Längsachse imaginären Axialverschiebung bei einer Vollumdrehung entspricht.

Die Drahtelemente 11, 12 sind im Allgemeinen spiralförmig um die Rotationsachse R gewunden, wobei die Drahtelemente 11, 12 in gleicher Richtung oder gegenläufig entlang der Rotationsachse R angeordnet sein können. Das einzelne Drahtelement 11, 12 weist demnach im Wesentlichen eine federförmige Struktur auf, wobei die Steigung des spiralförmig gewundenen Drahtelements 11 gegenüber dem Drahtelement 12 aufgrund der unterschiedlichen Winkel α', α" verschieden ist. Die Drahtelemente 11, 12 selbst können einen runden oder eckigen Querschnitt aufweisen. Zumindest an den Enden der Gitterstruktur 10 können die Drahtelemente 11, 12, insbesondere die freien Drahtenden 11', 12', miteinander verbunden, beispielsweise formschlüssig verbunden sein. Beispielsweise können die Drahtelemente 11, 12 miteinander verschweißt oder verklebt sein. Die hier beschriebenen Drahtelemente 11, 12 bilden die Gitterstruktur 10, d.h. die Drahtelemente 11, 12 sind derart spiralförmig auf den Umfang der Gitterstruktur angeordnet, dass der Querschnittsdurchmesser der gebildeten Spirale dem Querschnittsdurchmesser der Gitterstruktur 10 entspricht. Das Lumen der medizinischen Vorrichtung wird demnach durch den Querschnittsdurchmesser der einzelnen Drahtspiralen 11, 12 festgelegt.

Die Erfindung ist nicht auf streng rotationssymmetrische Gebilde eingeschränkt. Vielmehr umfasst die Erfindung Implantate oder medizinische Vorrichtungen wie Katheter oder Stabilizer, deren Wandung ein Innenlumen bildet bzw. einen Innenraum, wobei die Wandung auf die mit dieser im Gebrauch in Kontakt kommende Gefäßwand eine nach außen gerichtete Radialkraft ausübt. Dies ist beispielsweise auch bei einem Körper möglich, der einen hohlovalen oder anderen Querschnitt aufweist. Ferner genügt es, wenn das Implantat bzw. die medizinische Vorrichtung zumindest abschnittsweise rotationssymmetrisch bzw. annähernd rotationssymmetrisch ausgebildet ist, wobei weitere Abschnitte des Implantats bzw. der medizinischen Vorrichtung nicht rotationssymmetrisch ausgebildet sein können.

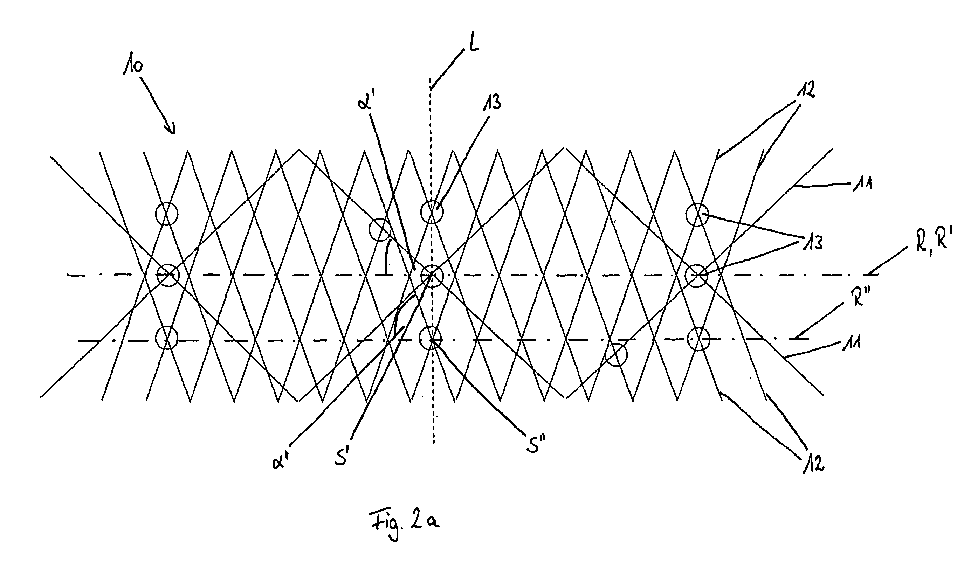

Fig. 2a zeigt ein weiteres Ausführungsbeispiel der erfindungsgemäßen medizinischen Vorrichtung, die mehrere die Gitterstruktur 10 bildende Drahtelemente 11, 12 umfasst. Die Gitterstruktur 10 ist dabei ebenfalls im aufgeklappten Zustand dargestellt, so dass die gesamte Umfangsfläche der Gitterstruktur 10 in der Zeichnungsebene abgebildet ist. Die Gitterstruktur 10 weist zwei erste Drahtelemente 11 auf, die insgesamt vier zweite Drahtelemente 12 überlagern. Eine andere Anzahl von ersten und zweiten Drahtelementen 11, 12 ist möglich. Die ersten Drahtelemente 11 weisen dabei einen kleineren Flechtwinkel α' auf als die zweiten Drahtelemente 12, wobei die Rotationsachse R der Gitterstruktur 10 gemäß Fig. 2a horizontal in der Zeichnungsebene angeordnet ist. In Fig. 2a sind ferner mehrere Kreuzungsbereiche 13 durch kreisförmige Markierungen hervorgehoben, in denen sich ein erstes Drahtelement 11 mit einem zweiten Drahtelement 12 überschneidet. Es können sich auch mehrere Drahtelemente 11, 12 in einem Kreuzungsbereich 13 überschneiden. Ferner ist eine Ebene L dargestellt, die durch drei Kreuzungsbereiche 13 verläuft, in denen sich jeweils zwei erste Drahtelemente 11 oder zwei zweite Drahtelemente 12 kreuzen. Die Schnittpunkte S', S" zwischen der Ebene L und den Drahtelementen 11, 12 sind in diesem Fall in den Kreuzungsbereichen 13 angeordnet. Der Schnittpunkt S' zwischen den beiden ersten Drahtelementen 11 und der Ebene L liegt dabei auf der Geraden R', die in der Darstellung gemäß Fig. 2a identisch mit der Rotationsachse R ist bzw. die Rotationsachse R überlagert. Die zweite Gerade R" ist parallel zur Rotationsachse R angeordnet und verläuft durch den zweiten Schnittpunkt S", der durch die Ebene L und wenigstens ein, hier zwei, zweite Drahtelemente 12 gebildet ist. In dem Fall, dass die Ebene L durcheinen Kreuzungsbereich 13 eines ersten Drahtelements 11 und eines zweiten Drahtelements 12 verläuft, fallen der erste Schnittpunkt S' und der zweite Schnittpunkt S" sowie die beiden Geraden R', R" zusammen bzw. sind identisch. Die zwischen den Geraden R', R" und den jeweils zugeordneten Drahtelementen 11, 12 gebildeten Winkel α', α" weisen unterschiedliche Werte auf. Insbesondere sind die zweiten Drahtelemente 12 unter einem größeren Flechtwinkel angeordnet als die ersten Drahtelemente 11, d.h. die durch die ersten Drahtelemente 11 gebildeten Drahtspiralen weisen eine größere Steigung auf als die durch die zweiten Drahtelemente 12 gebildeten Drahtspiralen. Grundsätzlich sind die Drahtelemente 11, 12 auf demselben Umfang der Gitterstruktur 10 angeordnet, d.h. die durch die Drahtelemente 11, 12 gebildeten spiralförmigen Rotationskörper weisen im Wesentlichen denselben Querschnittsdurchmesser auf. Dabei können die Drahtelemente 11, 12 miteinander verflochten bzw. verwoben sein oder sich überlagern. Beispielsweise ist ein erstes Drahtelemente 11 auf dem Außenumfang eines zweiten, spiralförmig gewundenen Drahtelements 12 angeordnet, wobei das erste Drahtelement 11 dieselbe Rotationsachse R aufweist wie das zweite Drahtelement 12.

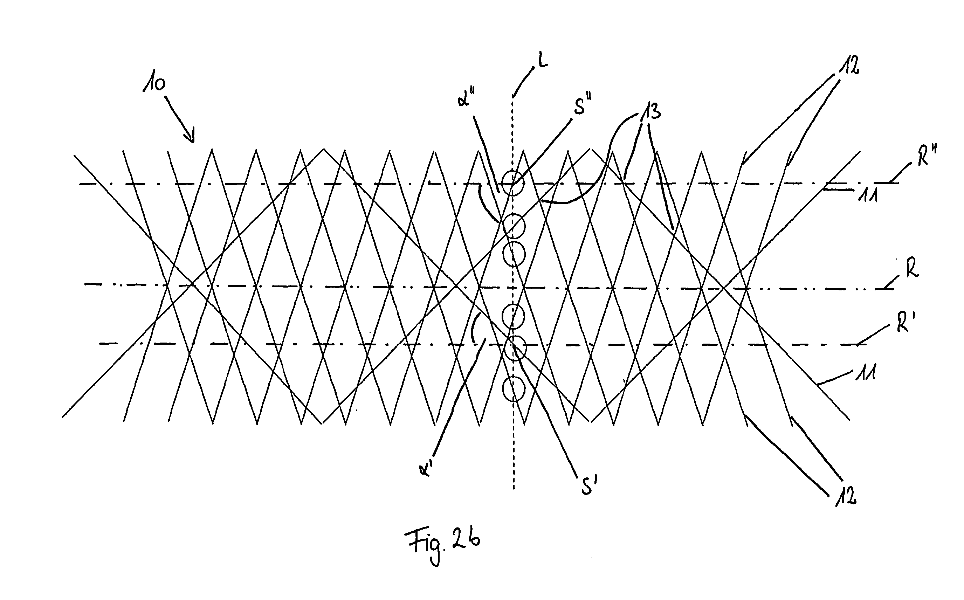

Fig. 2b zeigt im Wesentlichen dieselbe Gitterstruktur 10 wie Fig. 2a, wobei die Betrachtungsebene L gegenüber der Darstellung gemäß Fig. 2a in axialer Richtung versetzt angeordnet ist. Dabei ist die Betrachtungsebene L derart angeordnet, dass die Ebene L keinen Kreuzungsbereich 13 durchläuft. Die Schnittpunkte S', S" werden daher jeweils von nur einem Drahtelement 11, 12 und der Ebene L gebildet. In Fig. 2b sind die Schnittpunkte S', S" durch kreisförmige Markierungen hervorgehoben. Die Winkel α', α" zwischen den Drahtelementen 11, 12 und den parallel zur Rotationsachse R angeordneten Geraden R', R" sind ebenfalls unterschiedlich. Aus den Fig. 2a und 2b ist ersichtlich, dass die unterschiedlichen Winkel α', α" unabhängig von der Lage der Betrachtungsebene L erkennbar sind. Für die Bestimmung der Winkel α', α" ist allerdings die Anordnung der Referenzgeraden R', R" relevant, d.h. die Betrachtung der spitzen Winkel zwischen den Drahtelementen 11, 12 und der jeweiligen Projektion der Rotationsachse R auf die Umfangsebene im Schnittpunkt S', S".

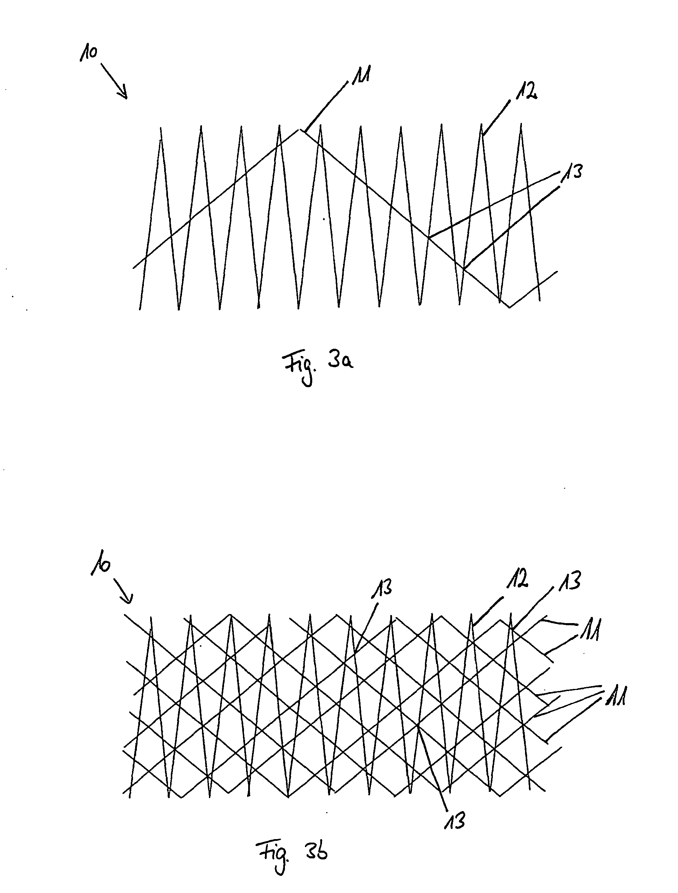

Fig. 3a zeigt ein weiteres Ausführungsbeispiel der erfindungsgemäßen medizinischen Vorrichtung, wobei die Gitterstruktur 10 aus zwei Drahtelementen 11, 12 gebildet ist, die einen unterschiedlichen Winkel α', α" aufweisen. Das erste Drahtelement 11 überlagert dabei das zweite Drahtelement 12. In den Kreuzungsbereichen 13 berühren sich die Drahtelemente 11, 12, wobei das erste Drahtelement 11 sowohl vollständig oberhalb des zweiten Drahtelements 12, als auch vollständig unterhalb des zweiten Drahtelements 12 verlaufen kann. Es ist außerdem möglich, dass das erste Drahtelement 11 das zweite Drahtelement 12 teilweise oberhalb und teilweise unterhalb kreuzen kann, insbesondere derart, dass das erste Drahtelement 11 mit dem zweiten Drahtelement 12 verflochten ist.

Ein weiteres Ausführungsbeispiel zeigt Fig. 3b, wobei das Ausführungsbeispiel im Wesentlichen eine Erweiterung der Gitterstruktur 10 gemäß Fig. 3a darstellt. Dabei überlagert eine Vielzahl von ersten Drahtelementen 11 ein einzelnes zweites Drahtelement 12. Das zweite Drahtelement 12 kann auch ein oder mehrere erste Drahtelemente 11 überlagern oder mit den ersten und/oder weiteren zweiten Drahtelementen 11, 12 verflochten sein. Die mehreren ersten Drahtelemente 11 können sich ebenfalls überlagern oder miteinander verflochten sein.

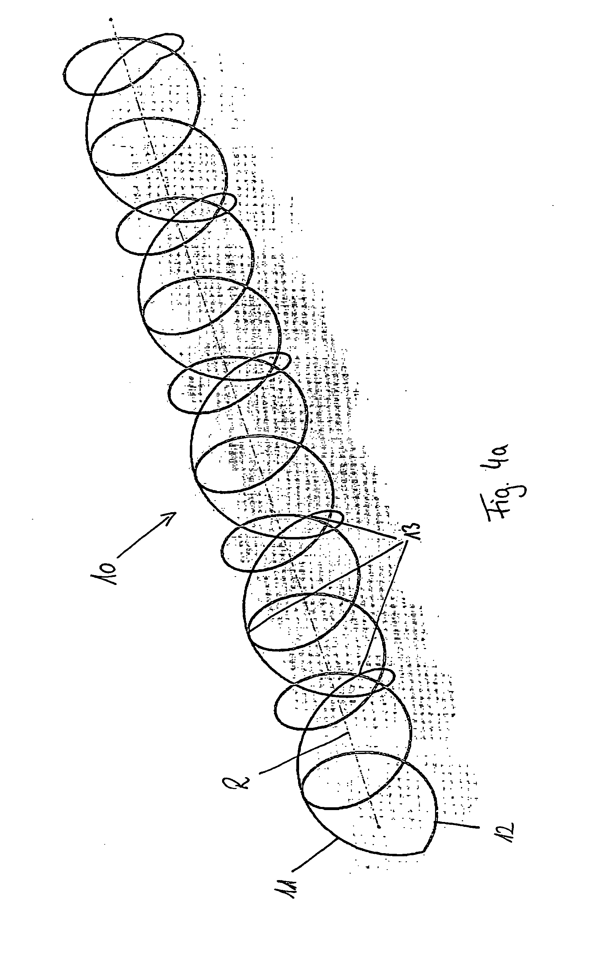

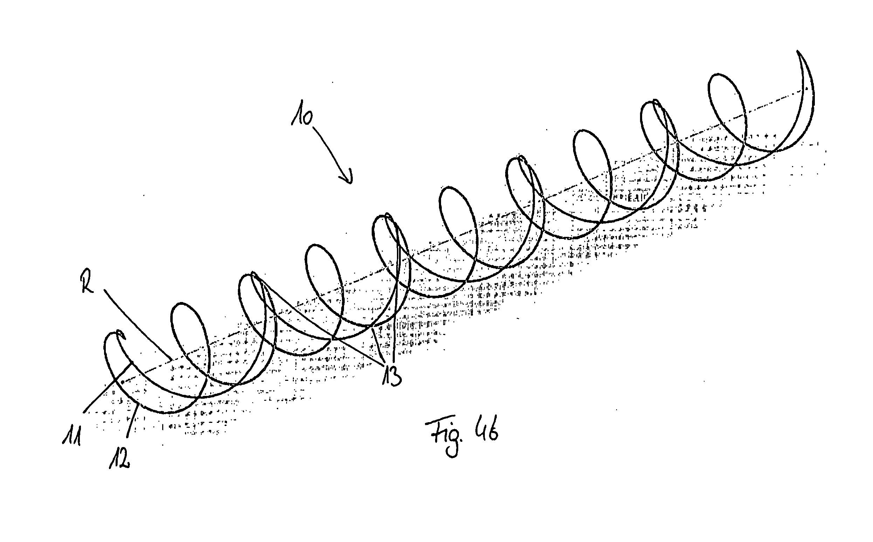

Die Fig. 4a und 4b zeigen ein Ausführungsbeispiel der erfindungsgemäßen medizinischen Vorrichtung mit einer Gitterstruktur 10, die aus zwei Drahtelementen 11, 12 gebildet ist. Dabei weist das erste Drahtelement 11 einen kleineren Winkel α' bezüglich der Rotationsachse R auf als das zweite Drahtelement 12. In der perspektivischen Darstellung gemäß den Fig. 4a und 4b ist der Winkelunterschied zwischen den Drahtelementen 11, 12 durch die unterschiedliche Steigung der spiralförmigen Drahtelemente 11, 12 erkennbar. Insbesondere ist der Winkelunterschied auch dadurch ersichtlich, dass das Drahtelement 11 bei einer Rotation um 360° um die Rotationsachse R eine größere axiale Längserstreckung entlang der Rotationsachse R aufweist als eine Rotation bzw. Windung des zweiten Drahtelements 12. An den axialen Enden der Gitterstruktur 10 sind die Drahtelemente 11, 12 miteinander verbunden. Es ist möglich, dass die beiden Drahtelemente 11, 12 aus einem einzigen Draht gebildet sind bzw. an den axialen Enden der Gitterstruktur 10 derart stoffschlüssig verbunden sind, dass die Gitterstruktur 10 im Wesentlichen aus einem einzigen durchgehenden Draht gebildet ist. Bei dem Ausführungsbeispiel gemäß Fig. 4a sind die Drahtelemente 11, 12 am axialen Ende der Gitterstruktur unter einem stumpfen Winkel verbunden, so dass die durch die Drahtelemente 11, 12 gebildeten Drahtspiralen im Wesentlichen gegenläufig angeordnet sind. Hingegen bildet die Verbindung zwischen den Drahtelementen 11, 12 am axialen Ende der Gitterstruktur 10 gemäß Fig. 4b einen spitzen Winkel, d.h. die Drahtelemente 11, 12 sind entlang der Rotationsachse R der Gitterstruktur 10 im Wesentlichen in gleicher Richtung angeordnet, wobei die Drahtspiralen unterschiedliche Steigungen aufweisen. Die Drahtelemente 11, 12 können allgemein an den axialen Enden der Gitterstruktur 10 bzw. des Stabilisierungsabschnitts formschlüssig verbunden sein. Die Drahtelemente 11, 12 können Abschlussknoten bilden. Andere Verbindungsarten sind möglich. Die Drahtelemente 11, 12 können auch in einem mittleren Abschnitt der Gitterstruktur 10 verbunden bzw. zueinander fixiert sein.

Ferner kann die Gitterstruktur 10 durch die Geflechtgeometrie fixiert sein, so dass eine Verbindung der Drahtelemente 11, 12 in den axialen Endabschnitten nicht erforderlich ist. Die reibschlüssige Verbindung der sich überlagernden bzw. überlappenden oder miteinander verflochtenen Drahtelemente 11, 12 in den Kreuzungsbereichen 13 bzw. Knoten ist zur Fixierung der Gitterstruktur 10 ausreichend. Bei einer Gitterstruktur 10, die einen Stabilisierungsabschnitt und zumindest einen weiteren Abschnitt aufweist, kann daher ein Übergangsbereich zwischen dem Stabilisierungsabschnitt und dem weiteren Abschnitt einen kontinuierlichen Übergang bilden. Die Drahtelemente 11, 12 können im Übergangsbereich zueinander lose bzw. nur reibschlüssig verbunden sein. Die asymmetrische Konfiguration der Gitterstruktur 10 wird dabei unverändert beibehalten.

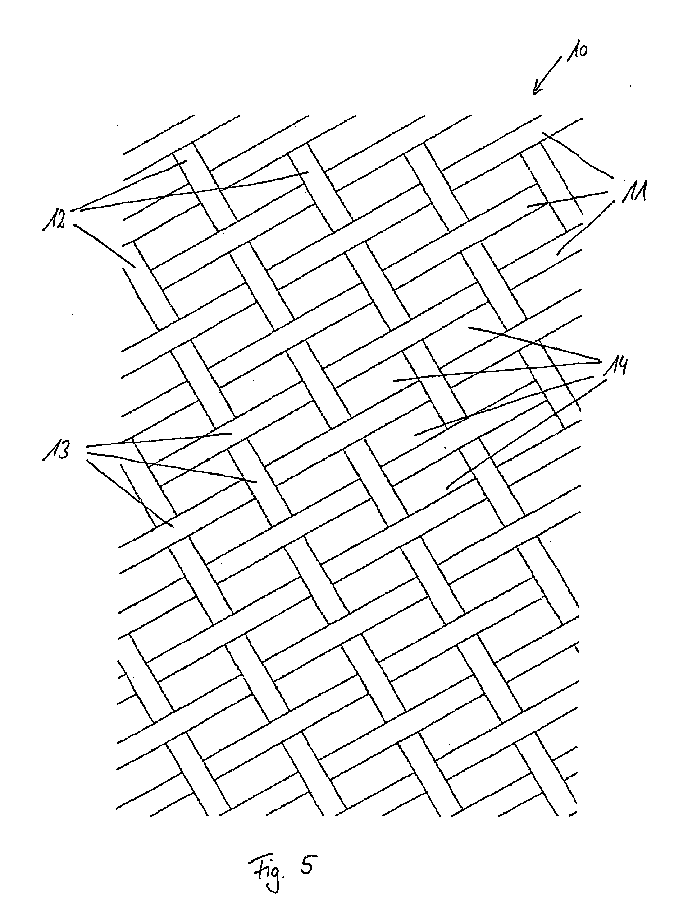

Eine Detailansicht einer asymmetrischen Flechtkonfiguration gemäß einem weiteren Ausführungsbeispiel der erfindungsgemäßen medizinischen Vorrichtung zeigt Fig. 5. Das dargestellte Gittergeflecht bzw. die Gitterstruktur 10 weist mehrere erste Drahtelemente 11 und mehrere zweite Drahtelemente 12 auf, wobei die ersten Drahtelemente 11 in der Zeichnungsebene diagonal von links unten nach rechts oben und die zweiten Drahtelemente 12 diagonal von rechts unten nach links oben verlaufen. Die Rotationsachse R (nicht dargestellt) verläuft in Fig. 5 vertikal in der Zeichnungsebene.

Die Asymmetrie, d.h. die unterschiedlichen Flechtwinkel der Drahtelemente 11, 12 ist dadurch erkennbar, dass die geflochtenen Drahtelemente 11, 12 keine rautenförmigen oder quadratischen Zellen 14 bilden. Vielmehr bilden die Drahtelemente 11, 12 rechteckförmige Zellen 14, deren Diagonalen nicht senkrecht, sondern zueinander angeordnet sind. Andere geometrische Ausgestaltungen der Zellen 14 sind möglich.

Die Drahtelemente 11, 12 sind ferner miteinander verflochten, wobei jedes erste Drahtelement 11 jeweils einmal unter und einmal über ein zweites Drahtelement 12 geführt ist. Andere Flechtkonfigurationen sind ebenfalls möglich, beispielsweise können zwei oder mehrere erste Drahtelemente 11 über und unter ein oder mehrere Drahtelemente 12 geführt werden.

Aus den Fig. 2a und 2b ist ersichtlich, dass die asymmetrische Flechtkonfiguration durch die Überlagerung wenigstens zweier in sich symmetrischer Unterstrukturen bzw. Geflechte erfolgt. Bei dem Ausführungsbeispiel gemäß Fig. 2 umfasst die erste Struktur 15 zwei erste Drahtelemente 11, die denselben Flechtwinkel α' aufweisen und zueinander symmetrisch angeordnet sind. Die erste Unterstruktur 15 ist daher in sich symmetrisch aufgebaut. Die zweite Unterstruktur 16 umfasst vier Drahtelemente 12, die jeweils denselben zweiten Flechtwinkel α" aufweisen. Die zweite Unterstruktur 16 ist daher ebenfalls in sich symmetrisch ausgebildet. Die jeweiligen Flechtwinkel α', α" der ersten Unterstruktur 15 und der zweiten Unterstruktur 16 sind voneinander verschieden. Insbesondere ist der erste Flechtwinkel α' der ersten Unterstruktur 15 kleiner als der zweite Flechtwinkel α" der zweiten Unterstruktur 16. Durch die Überlagerung bzw. Überlappung der beiden Unterstrukturen 15, 16 ergibt sich die asymmetrische Gesamtstruktur der Vorrichtung, die konkret daraus hervorgeht, dass die ersten und zweiten Drahtelemente 11, 12 jeweils unterschiedliche Flechtwinkel α' α" aufweisen.

Dasselbe gilt für die Vorrichtung gemäß Fig. 2b, die sich von der Vorrichtung gemäß Fig. 2a im Wesentlichen nur durch die Lage der imaginären Ebene L sowie der imaginären Geraden R', R" unterscheidet.

Es ist auch möglich, dass wenigstens zwei verschiedene Unterstrukturen mit unterschiedlichen Flechtwinkeln miteinander kombiniert sind bzw. zusammenwirken. Die Erfindung ist nicht auf die Kombination zweier in sich symmetrischer Unterstrukturen beschränkt, sondern umfasst die Kombination von mehr als zwei Unterstrukturen, beispielsweise von drei, vier oder mehr Unterstrukturen. Die einzelnen Unterstrukturen sind jeweils in sich symmetrisch mit demselben Flechtwinkel aufgebaut. Die Flechtwinkel der einzelnen Strukturen sind verschieden, so dass beispielsweise ein erster, zweiter, dritter oder vierter Flechtwinkel vorliegt. Die Flechtwinkel der weiteren (mehr als zwei) Strukturen können auch gleich groß sein. Verschiedenen Strukturen können in axialer Längsrichtung der Vorrichtung nachgeordnet sein und eine oder mehrere Strukturen überlagern, wobei die Flechtwinkel der sich jeweils überlagernden Strukturen unterschiedlich sind, so dass die Eigenschaften der Vorrichtung bzw. des Stents, beispielsweise im Hinblick auf die Steifigkeit, die Radialkraft, die Compliance usw. variabel sind.

Es ist auch möglich, dass die medizinische Vorrichtung, insbesondere das Gittergeflecht 10, teilweise ein asymmetrisches Geflecht aufweist. Beispielsweise kann die Gitterstruktur 10 einen Stabilisierungsabschnitt umfassen, der in einem Mittelbereich der Gitterstruktur 10 angeordnet ist. Die Randbereiche der Gitterstruktur 10 können ein symmetrisches Geflecht umfassen. Eine derartige Gitterstruktur 10 ist beispielsweise besonders zum Einsatz als künstliches Gefäß, d.h. Graft, einsetzbar, da sich der symmetrische Abschnitt der Gitterstruktur 10 in den Randbereichen ausdehnen und somit im Gefäß verankern kann, wohingegen im Mittelbereich der Gitterstruktur 10 auch bei hohen Druckschwankungen innerhalb des Gefäßes eine ausreichende Stabilität gewährleistet wird.

Im Allgemeinen wird durch einen großen Winkelunterschied zwischen den Drahtelementen 11, 12 eine hohe Stabilität der Gitterstruktur 10 erreicht. Die Stabilität in axialer Richtung wird dabei von einem Drahtelement 11, 12 bewirkt, das einen relativ flachen bzw. kleinen Winkel α', α"' zur Rotationsachse R aufweist, wohingegen die radiale Stabilität durch Erhöhung des Winkels α', α" eines weiteren Drahtelements 11, 12 bewirkt wird. Durch die Kombination zweier Drahtelemente 11, 12, wobei ein erstes Drahtelement 11 einen kleineren oder größeren Winkel α' aufweist als ein zweites Drahtelement 12, werden sowohl die axiale, als auch radiale Stabilität der Gitterstruktur 10 erhöht.

Die Drahtelemente 11, 12 bzw. alle die Gitterstruktur bildenden Drahtelemente weisen dasselbe Material auf, um die hohe Stabilität der Gitterstruktur 10 zu erreichen. Für den Einsatz der Vorrichtung als Stent oder künstliches Gefäß bzw. Gefäßprothese ist ein pseudoelastisches Material, beispielsweise Nitinol, geeignet. Das Material ist so konditioniert, dass bei Körpertemperatur eine pseudoelastische Dehnung erfolgt. Durch die pseudoelastischen Eigenschaften wird trotz der asymmetrischen Flechtkonfiguration eine geringe Durchmesseränderung ermöglicht, so dass ein derartig ausgebildeter Stent oder Stent-Graft im implantierten Zustand eine Radialkraft auf das Gefäß aufbringen kann, die das Implantat ausreichend im Gefäß fixiert.

*Im Zusammenhang mit der Erfindung werden die Ausführungsformen mit folgenden Merkmalen offenbart:*
- *Medizinische Vorrichtung, bei der die Drahtelemente 11, 12 zumindest an einem axialen Ende oder in einem Kreuzungsbereich 13 der Gitterstruktur 10 miteinander verbunden, insbesondere form-, kraft- und*/*oder stoffschlüssig verbunden, insbesondere verklebt oder verschweißt, sind.*
- *Medizinische Vorrichtung, bei der der Kreuzungsbereich 13 den Stabilisierungsabschnitt begrenzt.*
- *Medizinische Vorrichtung, bei der die Drahtelemente 11, 12 ein Formgedächtnismaterial und*/*oder ein pseudoelastisches Material, insbesondere eine Nickel-Titan-Legierung, umfassen.*
- *Medizinische Vorrichtung, bei der die Drahtelementen 11, 12 einen Kunststoff, insbesondere Polyester, Polyamid, Polypropylen oder Polyethylen, insbesondere HDPE oder UHMWPE, umfassen.*
- *Medizinische Vorrichtung, bei der eine flexible Umhüllung, die sich zumindest abschnittsweise in Umfangsrichtung und*/*oder axialer Richtung entlang der Gitterstruktur 10 erstreckt.*
- *Medizinische Vorrichtung, bei der die flexible Umhüllung einen Kunststoff, insbesondere Polyurethan, Silikon oder Teflon, umfasst.*
- *Medizinische Vorrichtung, bei der mehrere erste Drahtelemente 11 den ersten Winkel α' aufweisen und untereinander eine erste symmetrische Struktur 15 bilden und mehrere zweite Drahtelemente 12 den zweiten Winkel α" aufweisen und untereinander eine zweite symmetrische Struktur 16 bilden, wobei die erste Struktur 15 und zweite Struktur 16 überlagert sind derart, dass die ersten und zweiten Drahtelementen 11, 12 mit unterschiedlichen ersten und zweiten Winkeln α', α" einander zugeordnet sind.*
- *Verfahren zum Herstellen einer medizinischen Vorrichtung, bei dem wenigstens zwei Drahtelemente 11, 12 spiralförmig um eine gemeinsame Rotationsachse gewunden werden derart, dass die Drahtelemente 11, 12 in zumindest einer senkrecht zur Rotationsachse R angeordneten Ebene L mit einer parallel zur Rotationsachse R verlaufenden Geraden R', R"jeweils unterschiedliche spitze Winkel α'*, *α" einschließen*.
- *Verfahren zum Herstellern einer medizinische Vorrichtung, bei dem die Drahtelemente 11, 12 mit einer Textilmaschine, insbesondere Spinn- oder Webmaschine, geflochten und*/*oder gewunden werden.*

### Bezugszeichenliste

- 10: Gitterstruktur
- 11: erstes Drahtelement
- 12: zweites Drahtelement
- 13: Kreuzungsbereich
- 14: Zelle
- 15: erste Struktur
- 16: zweite Struktur

- R: Rotationsachse
- R': erste Gerade
- R": zweite Gerade
- S': erster Schnittpunkt
- S": zweiter Schnittpunkt
- α': erster Winkel
- α": zweiter, Winkel
- U: Begrenzungslinie

## Patentansprüche

1. Medizinische Vorrichtung aufweisend eine rotationssymmetrische Gitterstruktur (10) mit wenigstens zwei spiralförmig um eine gemeinsame Rotationsachse R gewundenen Drahtelementen (11, 12), die mit einer gemeinsamen, senkrecht zur Rotationsachse angeordneten Ebene L jeweils einen ersten und zweiten Schnittpunkt S', S" bilden, wobei durch den ersten Schnittpunkt S' eine erste Gerade R' und durch den zweiten Schnittpunkt S" eine zweite Gerade R" verlaufen, die jeweils parallel zur Rotationsachse R angeordnet sind und mit jeweils einem der beiden Drahtelemente (11, 12) einen spitzen Winkel (α', α") einschließen,
**dadurch gekennzeichnet, dass**
die beiden Winkel (α', α") unterschiedlich sind.

2. Medizinische Vorrichtung nach Anspruch 1,
**dadurch gekennzeichnet, dass**
alle Drahtelemente (11, 12) der Gitterstruktur (10) dasselbe inelastische Material umfassen derart, dass die Gitterstruktur (10) im Wesentlichen eine starre Geometrie aufweist.

3. Medizinische Vorrichtung nach Anspruch 1,
**dadurch gekennzeichnet, dass**
alle Drahtelemente (11, 12) der Gitterstruktur (10) dasselbe elastische Material umfassen derart, dass die rotationssymmetrische Gitterstruktur (10) im Wesentlichen eine veränderbare Geometrie aufweist.

4. Medizinische Vorrichtung nach wenigstens einem der Ansprüche 1 bis 3,
**dadurch gekennzeichnet, dass**
der Winkelunterschied zwischen dem ersten Winkel (α') und dem zweiten Winkel (α") wenigstens 2°, insbesondere wenigstens 5°, insbesondere wenigstens 8°, insbesondere wenigstens 10°, insbesondere wenigstens 20°, insbesondere wenigstens 30°, insbesondere wenigstens 40°, insbesondere wenigstens 45°, insbesondere wenigstens 50°, insbesondere wenigstens 60°, insbesondere wenigstens 70°, insbesondere wenigstens 90°, beträgt.

5. Medizinische Vorrichtung nach wenigstens einem der Ansprüche 1 bis 4,
**dadurch gekennzeichnet, dass**
der Winkelunterschied zwischen dem ersten Winkel (α') und dem zweiten Winkel (α") höchstens 10°, insbesondere höchstens 8°, insbesondere höchstens 6°, insbesondere höchstens 4°, insbesondere höchstens 2°, beträgt.

6. Medizinische Vorrichtung nach wenigstens einem der Ansprüche 1 bis 5,
**dadurch gekennzeichnet, dass**
der erste Winkel (α') kleiner als 45°, insbesondere kleiner als 40°, insbesondere kleiner als 20°, und der zweite Winkel (α") größer als 2°, insbesondere größer als 3°, insbesondere größer als 5°, insbesondere größer als 7°, insbesondere größer als 45°, insbesondere größer als 50°, insbesondere größer als 70°, ist.

7. Medizinische Vorrichtung nach wenigstens einem der Ansprüche 1 bis 6,
**dadurch gekennzeichnet, dass**
das Verhältnis zwischen der Anzahl der ersten Drahtelemente (11) und der Anzahl der zweiten Drahtelemente (12) höchstens 1:1, insbesondere höchstens 1:2, insbesondere höchstens 1:4, insbesondere höchstens 1:6, insbesondere höchstens 1:8, insbesondere höchstens 1:12, insbesondere höchstens 1:24, beträgt.

8. Medizinische Vorrichtung nach wenigstens einem der Ansprüche 1 bis 7,
**dadurch gekennzeichnet, dass**
das erste Drahtelement (11) mit der ersten Geraden R' einen kleineren Winkel α' einschließt als das zweite Drahtelement (12) mit der zweiten Geraden R".

9. Medizinische Vorrichtung nach wenigstens einem der Ansprüche 1 bis 8,
**gekennzeichnet durch**
wenigstens einen Stabilisierungsabschnitt, der sich zumindest bereichsweise in axialer Richtung entlang der Gitterstruktur (10) erstreckt, wobei der erste Winkel (α') entlang des Stabilisierungsabschnitts stets größer oder stets kleiner als der zweite Winkel (α") ist.

10. Medizinische Vorrichtung nach Anspruch 9,
**dadurch gekennzeichnet, dass**
der Stabilisierungsabschnitt einen axial mittleren Abschnitt (16) und/oder axialen Endabschnitt (17) der Gitterstruktur (10) bildet.

11. Medizinische Vorrichtung Anspruch 9 oder 10,
**gekennzeichnet durch**
zwei Stabilisierungsabschnitte, die jeweils einen axialen Endabschnitt (17) der Gitterstruktur (10) bilden.

12. Medizinische Vorrichtung nach wenigstens einem der Ansprüche 1 bis 11,
**dadurch gekennzeichnet, dass**
der erste Winkel (α') und/oder der zweite Winkel (α") zumindest abschnittsweise entlang der Gitterstruktur (10), insbesondere entlang einer parallel zur Rotationsachse R verlaufenden Geraden R', R", variiert.

13. Medizinische Vorrichtung nach wenigstens einem der Ansprüche 1 bis 12,
**dadurch gekennzeichnet, dass**
die rotationssymmetrische Gitterstruktur (10) im Wesentlichen rohrförmig, insbesondere stentartig, ausgebildet ist.

14. Medizinische Vorrichtung nach wenigstens einem der Ansprüche 1 bis 13,
**dadurch gekennzeichnet, dass**
die Drahtelemente (11, 12) entlang der Gitterstruktur (10) zumindest abschnittsweise coilartig gewunden sind.

15. Medizinische Vorrichtung nach wenigstens einem der Ansprüche 1 bis 14,
**dadurch gekennzeichnet, dass**
die Drahtelemente (11, 12) entlang der Gitterstruktur (10) zumindest abschnittsweise miteinander verflochten sind.

## Claims

1. Medical device comprising a rotationally symmetrical lattice structure (10) with a least two wire elements (11, 12) which are wound in a spiral fashion around a common axis of rotation R and which each form a first and second intersection S', S" with a common plane L arranged perpendicularly to the axis of rotation, wherein a first straight line R' runs through the first intersection S' and a second straight line R" runs through the second intersection S", said straight lines each being arranged in parallel to the axis of rotation R and each encompassing an acute angle (α', α") with one of the two wire elements (11, 12), **characterised in that** the two angles (α', α") are different.

2. Medical device according to claim 1, **characterised in that** all the wire elements (11, 12) of the lattice structure (10) comprise the same non-elastic material so that the lattice structure (10) essentially has a rigid geometry.

3. Medical device according to claim 1 **characterised in that** all the wire elements (11, 12) of the grid structure (10) comprise the same material so that the rotationally symmetrical lattice structure (10) essentially has an unchangeable geometry.

4. Medical device according to at least one of claims 1 to 3 **characterised in that** the difference in the angle between the first angle (α') and the second angle (α") is at least 2°, more particularly at least 5°, more particularly at least 8°, more particularly at least 10°, more particularly at least 20°, more particularly at least 30°, more particularly at least 40°, more particularly at least 45°, more particularly at least 50°, more particularly at least 60°, more particularly at least 70°, more particularly at least 90°.

5. Medical device according to at least one of claims 1 to 4 **characterised in that** the difference in the angle between the first angle (α') and the second angle (α") is at most 10°, more particularly at most 8°, more particularly at most 6°, more particularly at most 2°.

6. Medical device according to at least one of claims 1 to 5 **characterised in that** the first angle (α') is smaller than 45°, more particularly smaller than 40°, more particularly smaller than 20° and the second angle is greater than 2°, more particularly greater than 3°, more particularly greater than 5°, more particularly greater than 7°, more particularly greater than 45°, more particularly greater than 50°, more particularly greater than 70°.

7. Medical device according to least one of claims 1 to 6 **characterised in that** the ratio between the number of first wire elements (11) and the number of second wire elements (12) is at most 1:1, more particularly at most 1:2, more particularly at most 1:4, more particularly at most 1:6, more particularly at most 1:8, more particularly at most 1:12, more particularly at most 1:24.

8. Medical device according to at least one of claims 1 to 7 **characterised in that** the first straight line R' encompasses a smaller angle α' with the first wire element (11) than the second element (12) with the second straight line R".

9. Medical device according to at least one of claims 1 to 8 **characterised by** at least one stabilisation section that at least in parts extends in the axial direction along the lattice structure (10) wherein the first angle (α') along the stabilisation section is always greater or smaller than the second angle (α").

10. Medical device according to claim 9 **characterised in that** the stabilisation section forms an axial central section (16) and/or axial end section (17) of the lattice structure (10).

11. Medical device according to claim 9 or 10 **characterised by** two stabilisation section which each form an axial end section (17) of the lattice structure (10).

12. Medical device according to at least one claims of 1 to 11 **characterised in that** the first angle (α') and/or the second angle (α") varies at least in sections along the lattice structure (10), more particularly along a straight line R', R" running in parallel to the axis of rotation R.

13. Medical device according to at least one of claims 1 to 12 **characterised in that** the rotationally symmetrical lattice structure (10) is essentially designed in a tubular, more particularly a stent-like manner.

14. Medical device according to at least one of claims 1 to 13 **characterised in that** the wire elements (11, 12) are wound in a coil-like manner along the lattice structure (10) at least in section.

15. Medical device according to at least one of claims 1 to 14 **characterised in that** the wire elements (11, 12) are interlaced with each other along the lattice structure (10), at least in sections.

## Revendications

1. Dispositif médical présentant une structure grillagée symétrique en rotation (10) comportant au moins deux éléments filaires (11, 12) enroulés en spirale autour d'un axe de rotation R commun, qui constituent respectivement avec un plan L commun disposé perpendiculairement à l'axe de rotation un premier et un second point de coupe S', S", sachant que s'étendent à travers le premier point de coupe S' une première droite R' et le second point de coupe S" une seconde droite R" qui sont disposées respectivement parallèlement à l'axe de rotation R et qui circonscrivent avec respectivement un des deux éléments filaires (11, 12) un angle aigu (α', α''), **caractérisé en ce que** les deux angles (α', α'') sont différents.

2. Dispositif médical selon la revendication 1, **caractérisé en ce que** tous les éléments filaires (11, 12) de la structure grillagée (10) contiennent le même matériau inélastique, de sorte que la structure grillagée (10) présente sensiblement une géométrie rigide.

3. Dispositif médical selon la revendication 1, **caractérisé en ce que** tous les éléments filaires (11, 12) de la structure grillagée (10) contiennent le même matériau élastique, de sorte que la structure grillagée (10) symétrique en rotation présente sensiblement une géométrie variable.

4. Dispositif médical selon au moins une des revendications 1 à 3, **caractérisé en ce que** la différence angulaire entre le premier angle (α') et le second angle (α'') est d'au moins 2°, en particulier au moins 5°, en particulier au moins 8°, en particulier au moins 10°, en particulier au moins 20°, en particulier au moins 30°, en particulier au moins 40°, en particulier au moins 45°, en particulier au moins 50°, en particulier au moins 60°, en particulier au moins 70°, en particulier au moins 90°.

5. Dispositif médical selon au moins une des revendications 1 à 4, **caractérisé en ce que** la différence angulaire entre le premier angle (α') et le second angle (α") est d'au plus 10°, en particulier au plus 8°, en particulier au plus 6°, en particulier au plus 4°, en particulier au plus 2°.

6. Dispositif médical selon au moins une des revendications 1 à 5, **caractérisé en ce que** le premier angle (α') est inférieur à 45°, en particulier inférieur à 40°, en particulier inférieur à 20° et le second angle (α") est supérieur à 2°, en particulier supérieur à 3°, en particulier supérieur à 5°, en particulier supérieur à 7°, en particulier supérieur à 45°, en particulier supérieur à 50°, en particulier supérieur à 70°.

7. Dispositif médical selon au moins une des revendications 1 à 6, **caractérisé en ce que** le rapport entre le nombre de premiers éléments filaires (11) et le nombre de seconds éléments filaires (12) est au plus de 1 à 1, en particulier est au plus de 1 à 4, en particulier au plus de 1 à 6, en particulier au plus de 1 à 8, en particulier au plus de 1 à 12, en particulier au plus de 1 à 24.

8. Dispositif médical selon au moins une des revendications 1 à 7, **caractérisé en ce que** le premier élément filaire (11) circonscrit avec la première droite R' un angle α' plus petit que le second élément filaire (12) avec la seconde droite R".

9. Dispositif médical selon au moins une des revendications 1 à 8, **caractérisé par** au moins une section de stabilisation qui s'étend du moins par endroits dans le sens axial le long de la structure grillagée (10), le premier angle (α') étant toujours supérieur ou toujours inférieur au second angle (α'') le long de la section de stabilisation.

10. Dispositif médical selon la revendication 9, **caractérisé en ce que** la section de stabilisation forme une section axialement centrale (16) et/ou une section terminale axiale (17) de la structure grillagée (10).

11. Dispositif médical selon la revendication 9 ou 10, **caractérisé par** deux sections de stabilisation qui forment respectivement une section terminale axiale (17) de la structure grillagée (10).

12. Dispositif médical selon au moins une des revendication 1 à 11, **caractérisé en ce que** le premier angle (α') et/ou le second angle (α'') varient du moins par endroits le long de la structure grillagée (10), en particulier le long d'une droite R', R" s'étendant parallèlement à l'axe de rotation R.

13. Dispositif médical selon au moins une des revendications 1 à 12, **caractérisé en ce que** la structure grillagée symétrique en rotation (10) est réalisée sensiblement en forme de tube, en particulier à la manière d'un stent.

14. Dispositif médical selon au moins une des revendications 1 à 13, **caractérisé en ce que** les éléments filaires (11, 12) sont enroulés du moins par endroits à la manière d'une bobine le long de la structure grillagée (10).

15. Dispositif médical selon au moins une des revendications 1 à 14, **caractérisé en ce que** les éléments filaires (11, 12) sont entrelacés les uns aux autres du moins par endroits le long de la structure grillagée (10).
